# EUROPEAN PATENT SPECIFICATION

(11) **EP 2 978 474 B1**
(45) Date of publication and mention of the grant of the patent: **28.11.2018**
(21) Application number: 14717265.4
(22) Date of filing: 10.03.2014
(51) Int. Cl.: A61M 5/14, A61M 5/145, A61M 5/162, A61M 39/22

(54) **FLUID CARTRIDGE FOR MEDICAL INFUSION DEVICE**
FLÜSSIGKEITSKARTUSCHE FÜR EINE MEDIZINISCHE INFUSIONSVORRICHTUNG
CARTOUCHE DE FLUIDE POUR UN DISPOSITIF MÉDICAL DE PERFUSION

(30) Priority: 29.03.2013 US 201361806470 P
(43) Date of publication of application: 03.02.2016
(73) Proprietor: Animas Corporation, West Chester, PA 19380 (US)
(72) Inventor: SERVANSKY, Daniel, Lawrenceville, Georgia 30046 (US); BAKER, Daniel L., Drexel Hill, Pennsylvania 19026 (US); SAULENAS, William G., Wayne, NewJersey 07470 (US)
(74) Representative: Carpmaels & Ransford LLP
(86) International application number: PCT/US2014/022543
(87) International publication number: WO 2014/159213

(56) References cited:
- EP-A1- 0 761 247
- EP-A1- 0 761 247
- WO-A2-91/09639
- WO-A2-91/09639
- GB-A- 743 839
- US-A- 4 084 588
- US-A- 4 084 588
- US-A- 4 671 408
- US-A- 4 671 408
- US-A- 5 490 841
- US-A- 5 490 841
- US-A- 5 496 288
- US-A- 5 496 288
- US-A- 5 647 849
- US-A- 5 647 849

## Description

### Cross-Reference To Related Applications

This application claims priority to United States Provisional Application No. 61/806,470 filed on March 29, 2013.

### BACKGROUND OF THE RELATED ART

The present invention relates, in general, to medical devices and, in particular, to fluid cartridges with protected luer connections for use with medical infusion devices.

The use of drug delivery devices for various types of drug therapy is becoming more common as the automated infusion of a drug may provide more reliable and more precise treatment to a patient.

Diabetes is a major health concern, as it can significantly impede on the freedom of action and lifestyle of persons afflicted with this disease. Typically, treatment of the more severe form of the condition, Type I (insulin-dependent) diabetes, requires one or more insulin injections per day, referred to as multiple daily injections. Insulin is required to control glucose or sugar in the blood, thereby preventing hyperglycemia that, if left uncorrected, can lead to ketosis. Additionally, improper administration of insulin therapy can result in hypoglycemic episodes, which can cause coma and death. Hyperglycemia in diabetics has been correlated with several long-term effects of diabetes, such as heart disease, atherosclerosis, blindness, stroke, hypertension, and kidney failure.

The value of frequent monitoring of blood glucose as a means to avoid or at least minimize the complications of Type I diabetes is well established. Patients with Type II (non-insulin-dependent) diabetes can also benefit from blood glucose monitoring in the control of their condition by way of diet and exercise. Thus, careful monitoring of blood glucose levels and the ability to accurately and conveniently infuse insulin into the body in a timely manner is a critical component in diabetes care and treatment.

To more effectively control diabetes in a manner that reduces the limitations imposed by this disease on the lifestyle of the affected person, various devices for facilitating blood glucose (BG) monitoring have been introduced. Typically, such devices, or meters, permit the patient to quickly, and with a minimal amount of physical discomfort, obtain a sample of their blood or interstitial fluid that is then analyzed by the meter. In most cases, the meter has a display screen that shows the BG reading for the patient. The patient may then dose with the appropriate amount, or bolus, of insulin. For many diabetics, this results in having to receive multiple daily injections of insulin. In many cases, these injections are self-administered.

Due to the debilitating effects that abnormal BG levels can have on patients, i.e., hyperglycemia, persons experiencing certain symptoms of diabetes may not be in a situation where they can safely and accurately self-administer a bolus of insulin. Moreover, persons with active lifestyles find it extremely inconvenient and imposing to have to use multiple daily injections of insulin to control their blood sugar levels, as this may interfere or prohibit their ability to engage in certain activities. For others with diabetes, multiple daily injections may simply not be the most effective means for controlling their BG levels. Thus, to further improve both accuracy and convenience for the patient, insulin infusion pumps have been developed. These insulin delivery devices require that a reservoir of fluid be available to be delivered to the patient via a conduit typically referred to as an infusion set. Various cartridge designs have historically been prone to disconnection of the lineset that connects the lineset to the drug reservoir, due to unprotected connection between the lineset and the cartridge that may leak or become displaced when stressed.

It is therefore desirable to provide a structure for connection of the lineset to the cartridge in a manner that improves the reliability of the connection and decreases the possibility of leakage.

US 4,671,408 discloses a clam-shell for enclosing a syringe body including a Luer connector. WO 91/09639, US 5,490,841, and EP 0761247 disclose clam-shells for enclosing a syringe needle.

The invention is defined by the subject-matter of independent claim 1.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:
**FIGURES 1A** and **1B** illustrate a side-plan view and cross-sectional view, respectively, of a cartridge, luer connection, and lineset according to the prior art.
**FIGURE 2A** illustrates a side-plan view of a cartridge with a protected luer connection, according to an embodiment of the present invention.
**FIGURE 2B** illustrates a cross-sectional view of a cartridge with a protected luer connection, according to an embodiment of the present invention.
**FIGURE 2C** illustrates a top-plan view of a cartridge with a protected luer connection, according to an embodiment of the present invention, along the line B-B of **FIG. 2A****.**
**FIGURE 3A** illustrates a side-plan view of a cartridge with a protected luer connection, according to an embodiment of the present invention.
**FIGURE 3B** illustrates a cross-sectional view of a cartridge with a protected luer connection, according to an embodiment of the present invention.
**FIGURE 3C** illustrates a top-plan view of a cartridge with a protected luer connection, according to an embodiment of the present invention, along the line B-B of **FIG. 3A****.**
**FIGURE 4A** illustrates a side-plan view of a cartridge with a protected luer connection, according to an exemplary embodiment.
**FIGURE 4B** illustrates a cross-sectional view of a cartridge with a protected luer connection, according to an exemplary embodiment.
**FIGURE 5A** illustrates a side-plan view of a cartridge with a protected luer connection where the luer connection protection sleeve is in an open position, according to an exemplary embodiment.
**FIGURE 5B** illustrates a cross-sectional view of a cartridge with a protected luer connection, according to an exemplary embodiment.
**FIGURE 5C** illustrates a side-plan view of a cartridge with a protected luer connection, as in **FIG. 5A****,** where the luer connection protection sleeve is in a closed position, according to an exemplary embodiment.
**FIGURE 6A** illustrates a plan view in perspective of a cartridge with a protected luer connection, where the protection sleeve is shown with two finger grips.
**FIGURE 6B** illustrates a plan view in perspective of a cartridge with a protected luer connection according to **FIG. 6A****,** where the protection sleeve has multiple finger grips.
**FIGURE 6C** illustrates a plan view in perspective of a cartridge with a protected luer connection according to FIG. 6A, where the protection sleeve has a knurled finger grip.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. In addition, as used herein, the terms "patient," "host," "user" and "subject" refer to any human or animal subject and are not intended to limit the devices or methods to human use, although use of the subject invention in a human patient represents a preferred embodiment.

The present invention relates to cartridges that are used in drug delivery devices, including but not limited to insulin pumps. For purposes of illustration, this specification will refer to the structure and use of cartridges that store a quantity of medication and are inserted into a drug delivery device, such as an insulin pump, so that the medication can be infused into a patient.

Insulin pumps are devices which are typically worn on the patient's body, either above or below their clothing. These relatively small, unobtrusive devices typically store a quantity of insulin in a replaceable cartridge and include a processing unit, a display screen, and input functions such as buttons or a keypad. Such pumps may include the ability to run multiple insulin delivery programs, such as basal and bolus programs, to eliminate the need for injections of insulin via needles and syringes, by providing medication via an infusion device that can be worn by the patient for an extended period of time, usually in the range of 1-3 days.

Patients using insulin pumps typically have the ability to program insulin delivery times and amounts into their pump's software, and enter their blood glucose (BG) values into the pump via a data input system to deliver boluses of insulin in response to their activities, such as exercise and meal intake. Alternatively, the BG meter and pump may be in communication to permit the meter to transmit the BG reading to the pump along with a recommended bolus value, or to permit the pump or user to determine the appropriate bolus of insulin, if any.

Most diabetics that use an insulin infusion device purchase their insulin separately from the cartridges that they insert into their infusion pump. In order to fill the cartridge, they insert a needle attached to the cartridge into an insulin vial and pull back on an extractor. Once filled, they insert the cartridge into the insulin pump and attach an infusion set to the cartridge. The portion of the infusion set that attaches to the cartridge is generally referred to as the luer. The luer connects to tubing, referred to as the lineset tubing, that terminates in a cannula that is inserted under the skin of the patient to permit the infusion of the insulin. The cannula is generally held in place with an adhesive patch, to avoid accidental dislodgement.

The cartridge, plunger and the extractor are typically formed from implantable grade plastic, such as long-term implantable plastics including, but not limited to polyethylenes, polyetheretherketones ("PEEK") and bioabsorbables-polylactic acid ("PLA"), polyglycolic acid ("PGA") and their copolymers. In instances where the materials do not need to be of an implantable grade, those skilled in the art will readily recognize that numerous additional plastics that are suitable for use, including various polyethylene and polyester acrylates and resins. Extrusion, injection molding, and casting are typical manufacturing methods employed for this process.

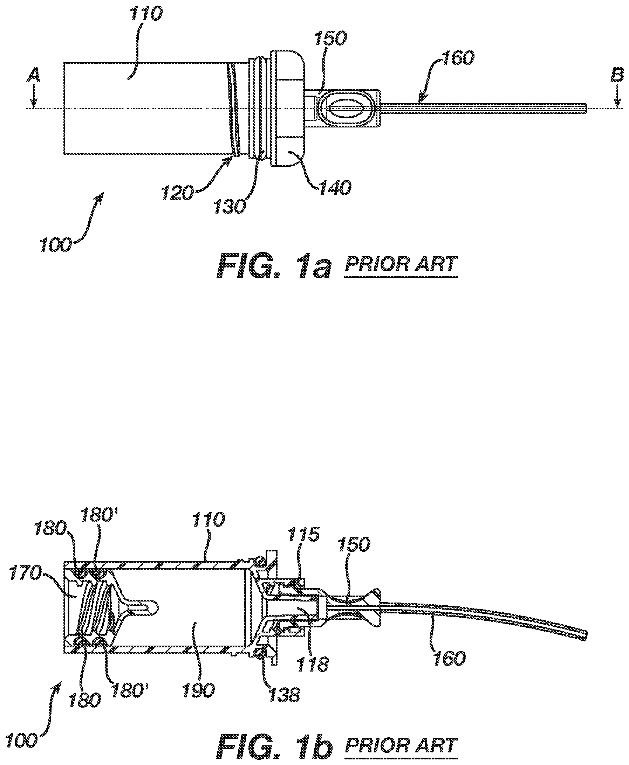

**FIGS. 1A and 1B** refers to a prior art device that is exemplary of cartridges presently used to store medication and use with an infusion device. The cartridge 100 has a body 110 with a reservoir 190 for storing fluid. The cartridge body 110 has an external thread 120 to allow the cartridge to be screwably secured within a cavity within the drug delivery device (not shown).

A plunger 170 is inserted into the reservoir 190 to expel the fluid out of the cartridge via a nipple 118. The head of the plunger 170 is generally equipped with one or more O-rings 180, 180' to minimize leakage from between the plunger 170 and the interior of the reservoir 190. An infusion set (partially shown) comprising a luer 150 and lineset tubing 160 is secured to the cartridge to permit fluid communication by screwing the luer 150 into a luer connector 115 that has internal threads for receiving the luer 150. Once attached, the luer is secured to the cartridge using a cartridge cap 140. An O-ring 130 is typically included to maintain a seal between the cartridge 100 and the cavity in which it is disposed within a drug infusion device.

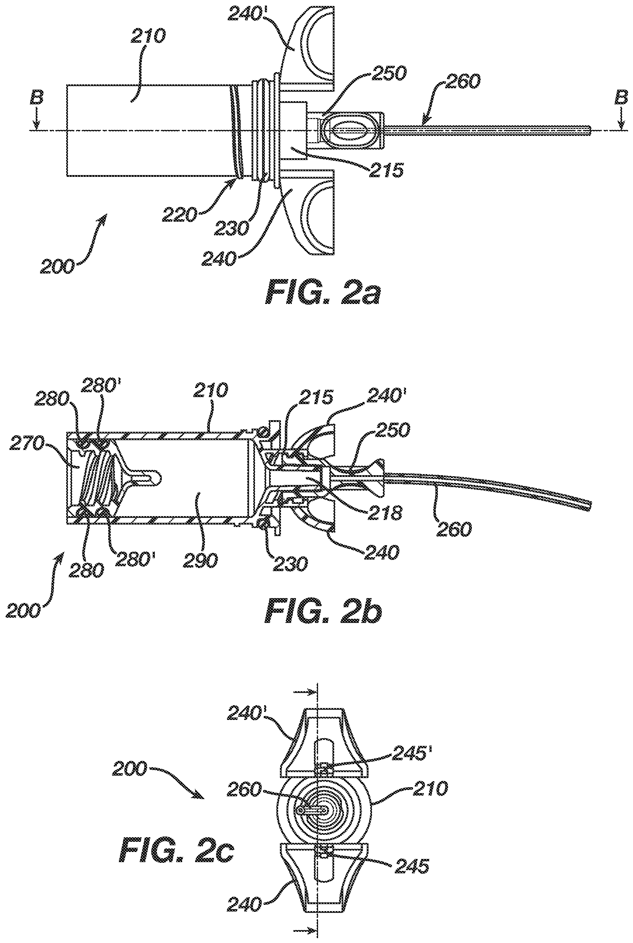

The prior art device leaves the luer 150 exposed and vulnerable to damage or breakage due to the manner in which it protrudes from the cartridge and extends beyond the body of the housing of the drug infusion device. An improved cartridge according to one embodiment of the present invention is illustrated in **FIGS. 2A-2C****.** The improved cartridge disclosed herein provides a protective cartridge cap integral to the cartridge that encloses the luer and luer connection, protecting it from damage or breakage due to shock or accidental contact.

As illustrated in **FIGS. 2A-2C** the cartridge 200 has a body 210 with a reservoir 290 for storing fluid. The cartridge body 210 has an external thread 220 to allow the cartridge to be screwably secured within a cavity within the drug delivery device (not shown). A plunger 270 is inserted into the reservoir 290 to expel the fluid out of the cartridge via a nipple 218. The head of the plunger 270 is generally equipped with one or more O-rings 280, 280' to minimize leakage from between the plunger 270 and the interior of the reservoir 290.

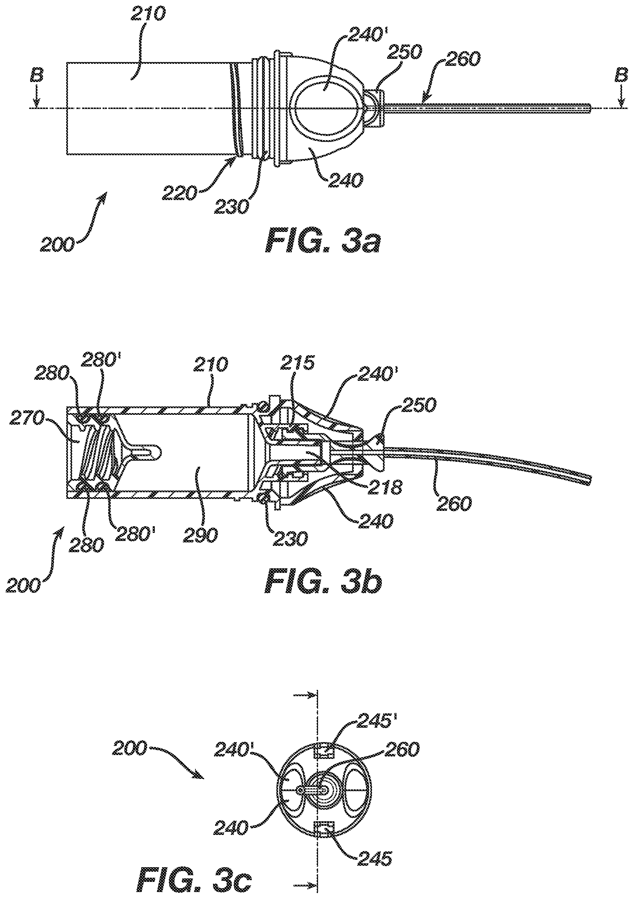

An infusion set (partially shown) comprising a luer 250 and lineset tubing 260 is secured to the cartridge to permit fluid communication by screwing the luer 250 into a luer connector 215 that has internal threads for receiving the luer 250. Once the luer is attached, the clam-shell style cap halves 240, 240' rotate via hinges 245, 245' from an open position as illustrated in **FIGS 2A-2C** into the closed position illustrated in **FIGS. 3A-3C** to form a protective housing around the luer 250 and luer connector 215. An O-ring 230 may be included to maintain a seal between the cartridge 200 and the cavity in which it is disposed within a drug infusion device.

The clam shell configuration of **FIGS. 2A-2C** and **3A-3C** utilizes two half-dome shells 240, 240' which either snap into hinges on the top of the cartridge or are molded with the cartridge through living hinges (not shown). When the clam shell is open it allows the user to access the luer 250 to connect the infusion lineset 260 to the cartridge 200. When the clam shell is closed around this fitting, it snaps into place with an eccentric locking tab which is located on the hinge (not shown). In the closed position, the clam shells form a structural barrier around the luer 250 and luer connector 215. Recessed pockets in the clam shells may be used to form finger grips to allow for easy threading and installation of the cartridge into an infusion pump. The clam shells may be manufactured of injection molded plastics such as acrylonitrile butadiene styrene ("ABS") for its strength and limited shrinkage during the molding process, polypropylene for its flexibility and application to living hinges, vinyl for its strength and durability, nylon for its strength and resistance to wear in the hinge application, or polycarbonate for its strength, rigidity and cost effectiveness. Other materials may be used, depending on the application.

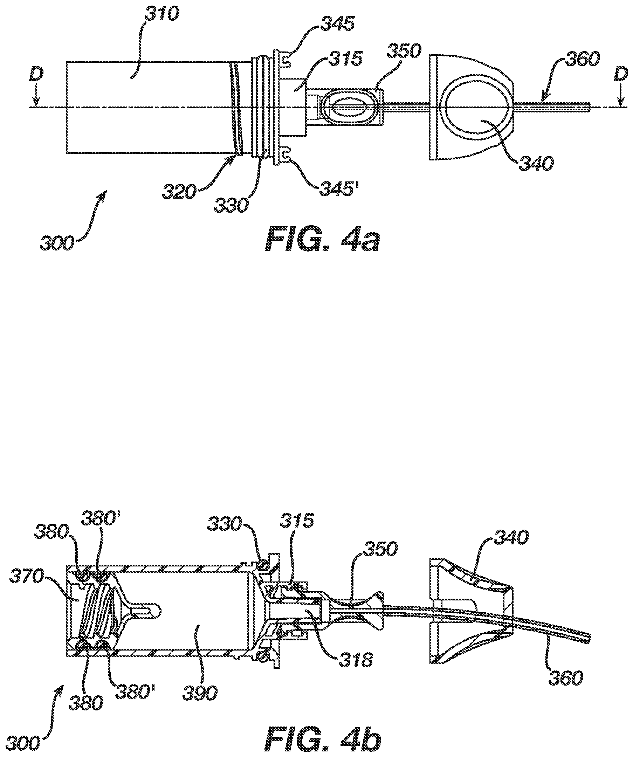

Another embodiment is illustrated in **FIGS. 4A-4B** in which the cartridge 300 has a body 310 with a reservoir 390 for storing fluid. The cartridge body 310 has an external thread 320 to allow the cartridge to be screwably secured within a cavity within the drug delivery device (not shown). A plunger 370 is inserted into the reservoir 390 to expel the fluid out of the cartridge via a nipple 318. The head of the plunger 370 is generally equipped with one or more O-rings 380, 380' to minimize leakage from between the plunger 370 and the interior of the reservoir 390.

An infusion set (partially shown) comprising a luer 350 and lineset tubing 360 is secured to the cartridge to permit fluid communication by screwing the luer 260 into a luer connector 315 that has internal threads for receiving the luer 360. Once attached, protective cap 340 is moved into place over the luer 350 and luer connector 315 and removably attached to the cartridge 300 by frictional forces using snap-in points 345, 345'. An O-ring 330 may be included to maintain a seal between the cartridge 300 and the cavity in which it is disposed within a drug infusion device.

The snap fit configuration utilizes one full-dome shell which snaps into snap-in points 345, 345' on the top of the cartridge 300. The user threads the infusion set luer 350 through the top of the cap 340 before connecting the infusion lineset 360 to the nipple 318 of the cartridge 300. Once the luer connection is made, the user snaps the cap into the snap-in points which locks the cap into place on top of the cartridge. In the closed position, the snap cap forms a structural barrier around the luer connection. Recessed pockets in the snap cap form finger grips to allow for easy threading and installation into the pump. The snap cap may be injection molded in plastics such as ABS for its strength and limited shrinkage during the molding process; polypropylene for its flexibility and application to living hinges; vinyl for its strength and durability; nylon for its strength and resistance to wear in the hinge application; or polycarbonate for its strength, rigidity and cost effectiveness.

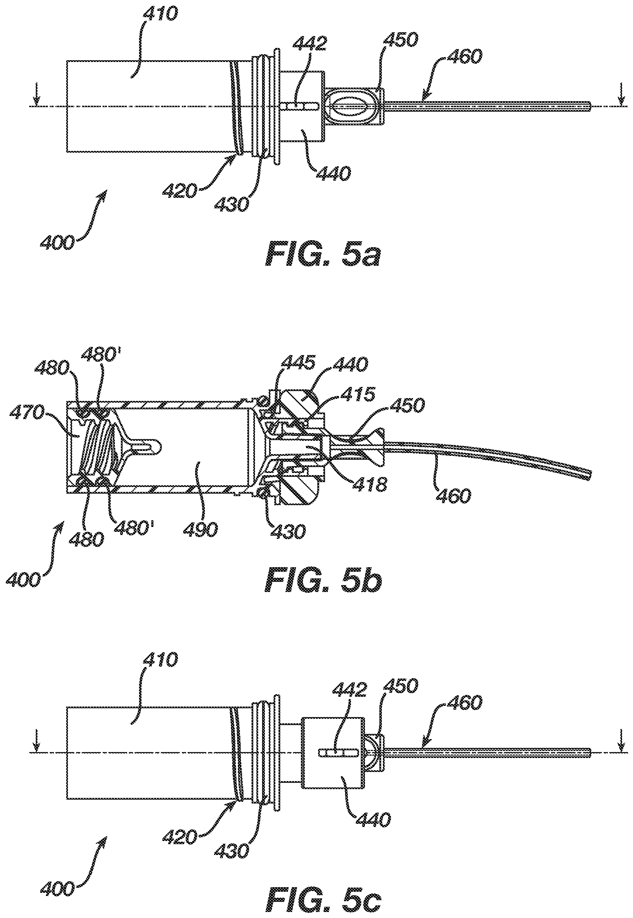

Another embodiment is illustrated in **FIGS. 5A-5C** in which the cartridge 400 has a body 410 with a reservoir 490 for storing fluid. The cartridge body 410 has an external thread 420 to allow the cartridge to be screwably secured within a cavity within the drug delivery device (not shown). A plunger 470 is inserted into the reservoir 490 to expel the fluid out of the cartridge via a nipple 418. The head of the plunger 470 is generally equipped with one or more O-rings 480, 480' to minimize leakage from between the plunger 470 and the interior of the reservoir 490.

An infusion set (partially shown) comprising a luer 450 and lineset tubing 460 is secured to the cartridge to permit fluid communication by screwing the luer 450 into a luer connector 415 that has internal threads for receiving the luer 450. Once the luer is attached to the luer connector, a telescoping sleeve 440 is slid into place over the luer 450 and luer connector 415 by moving the telescoping sleeve 440 away from the body of the cartridge 400 and creating a friction fit between the luer 450 and the telescoping sleeve 440. The friction fit is aided by frictional grooves 442 that permit a small portion of the luer to "lock" into the grooves. **FIG. 5A** shows the telescoping sleeve 440 in an open position that permits the luer 450 to be inserted into and secured to the luer connector. **FIG. 5C** shows the telescoping sleeve 440 in a closed position, where it frictionally secured to the luer 450, thereby protecting the luer 450 from breakage or damage and prohibiting the luer 450 from rotation and unintentional disconnection. An O-ring 430 may be included to maintain a seal between the cartridge 400 and the cavity in which it is disposed within a drug infusion device.

The telescoping configuration utilizes a cylindrical sleeve 440 which fits around the luer 450 on the cartridge 400. When in the retracted position, the user has access to the luer connection to connect the infusion lineset 460 to the nipple 418 of the cartridge 400. Once the connection is made, the user pulls the cylindrical sleeve 440 upward to cover the luer 450. The cylindrical sleeve 440 may be held in the upright position through a tab/recess snap (e.g. frictional grooves 442) in cylindrical sleeve. The cylindrical sleeve may have two or more finger grips, or wings, which act as grip points to help the user pull the cylindrical sleeve upward and then thread the cartridge 400 into an infusion pump. The cylindrical shell may be injection molded in plastics such as ABS for its strength and limited shrinkage during the molding process; polypropylene for its flexibility and application to living hinges; vinyl for its strength and durability; nylon for its strength and resistance to wear in the hinge application; or polycarbonate for its strength, rigidity and cost effectiveness.

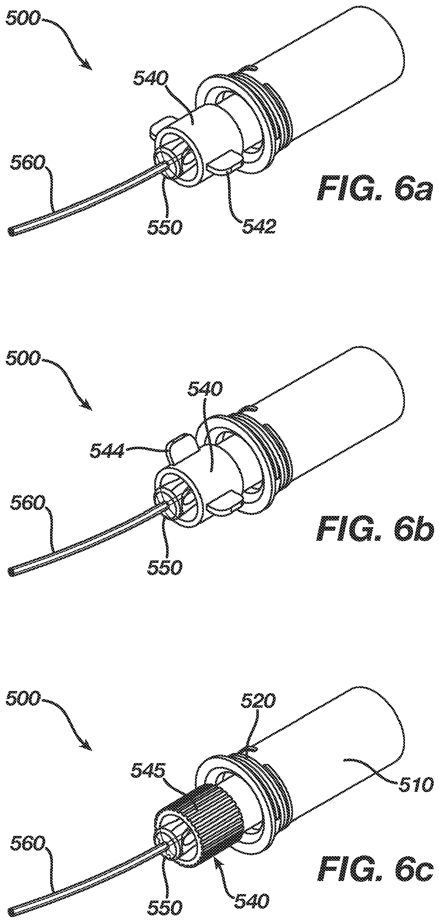

**FIGS 6A-6C** illustrates embodiments with differing designs for the telescoping sleeve 540. The cartridge 500 having body 510 and external thread 520 is attached to the luer 550 and lineset 560. **FIG. 6A** shows a telescoping sleeve 540 with two finger grips 542. **FIG. 6B** shows a telescoping sleeve 540 with three finger grips 544. Finally, **FIG. 6C** shows a telescoping sleeve 540 with a knurled finger grip 545.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that devices and methods within the scope of these claims and their equivalents be covered thereby.

## Claims

1. A medicament cartridge (200), comprising:
a housing (210) having an open proximal end, a distal end, and a cavity therein defining a reservoir (290);
a plunger (270) configured to slidably insert into the proximal end of the cartridge (200);
a luer connector (215) at the distal end of the cartridge (200), the luer connector (215) having internal threads and a nipple in fluid communication with the reservoir (290);
a two-piece clam-shell protective cover (240, 240') at the distal end of the housing (210);
a luer (250) configured for screwable attachment to the luer connector (215) ; and
a lineset (260) securable to the nipple by the screwable attachment of the luer (250) to the luer connector (215), to place the lineset (260) in fluid communication with the reservoir (290);
wherein the two-piece clam-shell protective cover (240, 240') comprises clam-shell style cap halves (240, 240') movable from an open position into a closed position to form a protective housing around the luer (250) and the luer connector (215) when the lineset (260) is in fluid communication with the reservoir (290) via the luer (250).

2. The cartridge (200) of claim 1 comprising an external thread (220) disposed on the housing (210).

3. The cartridge (200) of claim 1 wherein the plunger (270) includes one or more o-rings seals (230).

4. The cartridge (200) of claim 1 wherein each clam-shell style cap half (240, 240') comprises a half-dome shell, each half-dome shell rotatably attached to the housing (210).

5. The cartridge of claim 6 wherein each half-dome shell (240, 240') is rotatably attached to the housing (210) by a living hinge.

## Patentansprüche

1. Medikamentenkartusche (200), umfassend:
ein Gehäuse (210) mit einem offenen proximalen Ende, einem distalen Ende und einem Hohlraum, der ein Reservoir (290) darin definiert;
einen Stempel (270), der zum verschiebbaren Einführen in das proximale Ende der Kartusche (200) gestaltet ist;
ein Luer-Verbindungselement (215) an dem distalen Ende der Kartusche (200), wobei das Luer-Verbindungselement (215) ein Innengewinde und einen Stutzen in Fluidverbindung mit dem Reservoir (290) aufweist;
eine zweiteilige Muschelschalen-Schutzhülle (240, 240') an dem distalen Ende des Gehäuses (210);
einen Luer (250), der zur Schraubbefestigung an das Luer-Verbindungselement (215) ausgelegt ist; und
einen Leitungssatz (260), der durch die Schraubbefestigung des Luers (250) an das Luer-Verbindungselement (215) an dem Stutzen befestigbar ist, um den Leitungssatz (260) in Fluidverbindung mit dem Reservoir (290) zu bringen;
wobei die zweiteilige Muschelschalen-Schutzhülle (240, 240') muschelschalenartige Kappenhälften (240, 240') umfasst, die von einer offenen Stellung in eine geschlossene Stellung beweglich sind, um ein Schutzgehäuse um den Luer (250) und das Luer-Verbindungselement (215) zu bilden, wenn sich der Leitungssatz (260) über den Luer (250) in Fluidverbindung mit dem Reservoir (290) befindet.

2. Kartusche (200) gemäß Anspruch 1, umfassend ein Außengewinde (220), das an dem Gehäuse (210) angeordnet ist.

3. Kartusche (200) gemäß Anspruch 1, wobei der Stempel (270) eine oder mehrere O-Ring-Dichtungen (230) aufweist.

4. Kartusche (200) gemäß Anspruch 1, wobei jede muschelschalenartige Kappenhälfte (240, 240') eine halbkuppelförmige Schale umfasst, wobei jede halbkuppelförmige Schale an dem Gehäuse (210) befestigt ist.

5. Kartusche gemäß Anspruch 6, wobei jede halbkuppelförmige Schale (240, 240') über ein Filmscharnier drehbar an dem Gehäuse (210) befestigt ist.

## Revendications

1. Cartouche de médicament (200) comprenant :
un boîtier (210) ayant une extrémité proximale ouverte, une extrémité distale et une cavité à l'intérieur de celui-ci définissant un réservoir (290) ;
un piston (270) configuré pour s'insérer de manière coulissante dans l'extrémité proximale de la cartouche (200):
un raccord luer (215) au niveau de l'extrémité distale de la cartouche (200), le raccord luer (215) ayant des filetages internes et une tétine en communication fluidique avec le réservoir (290) ;
un couvercle de protection à double coque en deux parties (240, 240') au niveau de l'extrémité distale du boîtier (210) ;
un luer (250) configuré pour une fixation vissable au raccord luer (215) ;
un ensemble de tubes (260) pouvant être fixé à la tétine par la fixation vissable du luer (250) au raccord luer (215), pour placer l'ensemble de tubes (260) en communication fluidique avec le réservoir (290) ;
le couvercle de protection à double coque en deux parties (240, 240') comprenant des moitiés de capuchon de type à double coque (240, 240') mobiles d'une position ouverte à une position fermée pour former un boîtier de protection autour du luer (250) et du raccord luer (215) lorsque l'ensemble de tubes (260) est en communication fluidique avec le réservoir (290) par l'intermédiaire du luer (250).

2. Cartouche (200) selon la revendication 1, comprenant un filetage externe (220) disposé sur le boîtier (210).

3. Cartouche (200) selon la revendication 1, le piston (270) comprenant un ou plusieurs joints toriques (230).

4. Cartouche (200) selon la revendication 1, chaque moitié de capuchon de type à double coque (240, 240') comprenant une coque en demi-dôme, chaque coque en demi-dôme étant fixée rotative au boîtier (210).

5. Cartouche selon la revendication 6, chaque coque en demi-dôme (240, 240') étant fixée rotative au boîtier (210) par une charnière mobile.
